# EUROPEAN PATENT APPLICATION

(11) **EP 1 980 563 A1**
(43) Date of publication of application: **15.10.2008**
(21) Application number: 08251374.8
(22) Date of filing: 09.04.2008
(51) Int. Cl.: C07D 495/04

(54) **Procedure for the preparation of methyl (+)-(S)-Alpha-(O-chlorophenyl)-6,7-dihydrothieno-[3,2-C]pyridine-5(4H) acetate**

(30) Priority: 09.04.2007 IN CH07342007
(71) Applicant: BATTULA, Srinivasa Reddy, RA Chem Pharma Limited 6-3-1239/2 'Amar House Road, Somajiguda Hyderbad - 500 082, State of Andhra Pradesh (IN)
(72) Inventor: BATTULA, Srinivasa Reddy, RA Chem Pharma Limited 6-3-1239/2 'Amar House Road, Somajiguda Hyderbad - 500 082, State of Andhra Pradesh (IN)
(74) Representative: Watson, Robert James

(57) **Abstract**

A process for the preparation of Methyl (+)-(S)-α-(o-chlorophenyl)-6,7-dihydrothieno-[3,2-c]pyridine-5(4H)acetate of formula I: 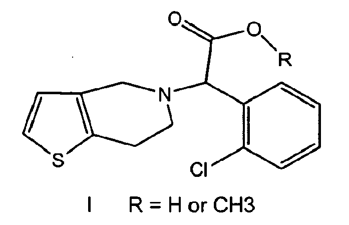 the process comprising:
a. reacting 4,5,6,7-tetrahydro[3,2-c]thieno pyridyl methane (II) with 2-halophenyl acetic acid derivative of formula III 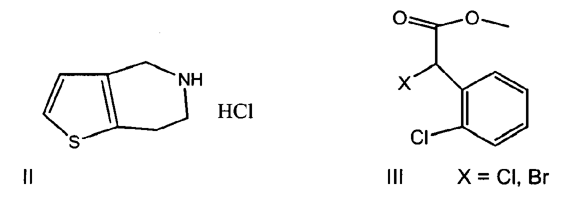 b. extracting the formed product (+/-) clopidogrel in to solvent from the reaction mixture of step a;
c. resolution of (+/-) clopidogrel of step b with levorotatory camphor sulfonic acid and isolation of the (+) clopidogrel camphor sulfonic acid salt;
d. purifying (+) clopidogrel camphor salt; and
e. neutralization of the camphor salt of step d with aqueous base in solvent and isolation of (+) clopidogrel base by separation and removal solvent.

## Description

### Technical Field

The present invention relates to the procedure for the preparation of thieno[3,2-c]pyridine derivative of formula I. 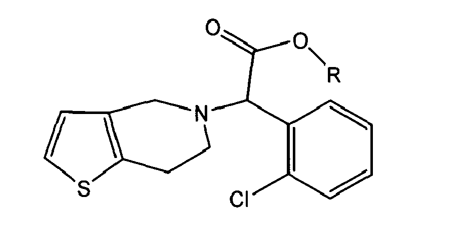 Formula I, where R = H or CH₃

Compounds of formula I known for the therapeutic value notable for its inhibition of platelet aggregation and antithrombic properties more particularly to a process for the preparation of S (+) clopidogrel

### Background

Pharmacologically active and significant anti aggregate and anti thrombic thieno[3,2-c pyridine derivative disclosed in FR2,215,948 , FR2530,247 ,FR2612929. Clopidogrel having anti platelet and anti thrombic activity first disclosed by EP0099802 and US4529596. It is synthesized by reaction between 4,5,6,7-tetrahydro[3,2-c]thienopyridine and 2-chloro-2-(2-chlorophenyl)acetate.

US 4529596, GB0420706 and GB0466569 reported methods to synthesize the compound of formula I using derivative of α-halo phenyl acetic acid with 4,5,6,7-tetrahydro thieno pyridine. The process involves multi step resulting over all low yields. US4847265 discloses that the biological activity is due to (s) -isomer in case of clopidogrel According to this process the product is obtained in a racemic mixture. EP0281459 discloses the separation of two enantiomers with optical resolution.
Various other procedures disclosed in US4876362, US5036156, US5132435, US5239170, US5204469, US6080875, WO98/51681, 682, 689 and W09918110.

Various other procedures disclosed in US4876362, US5036156, US5132435, US5239170, US5204469, US6080875, WO98/51681, 682, 689 and W09918110. WO98/51689 discloses the preparation of clopidogrel by reacting 2(2-thienyl)ethylamine with ortho chloro benzaldehyde and sodium cyanide resulting Nitrile is converted to amide followed by methyl ester and cyclised with formaldehyde to give clopidogrel. EP466569 discloses alternative method of reaction methyl(2-chlorophenyl)acetate with 2-(2thienyl)ethanol derivative where X is halogen or a sulfur group.

Most of the reported procedures for resolution of RS clopidogrel to useful (+) clopidogrel were reported with base liberated from suitable salt.

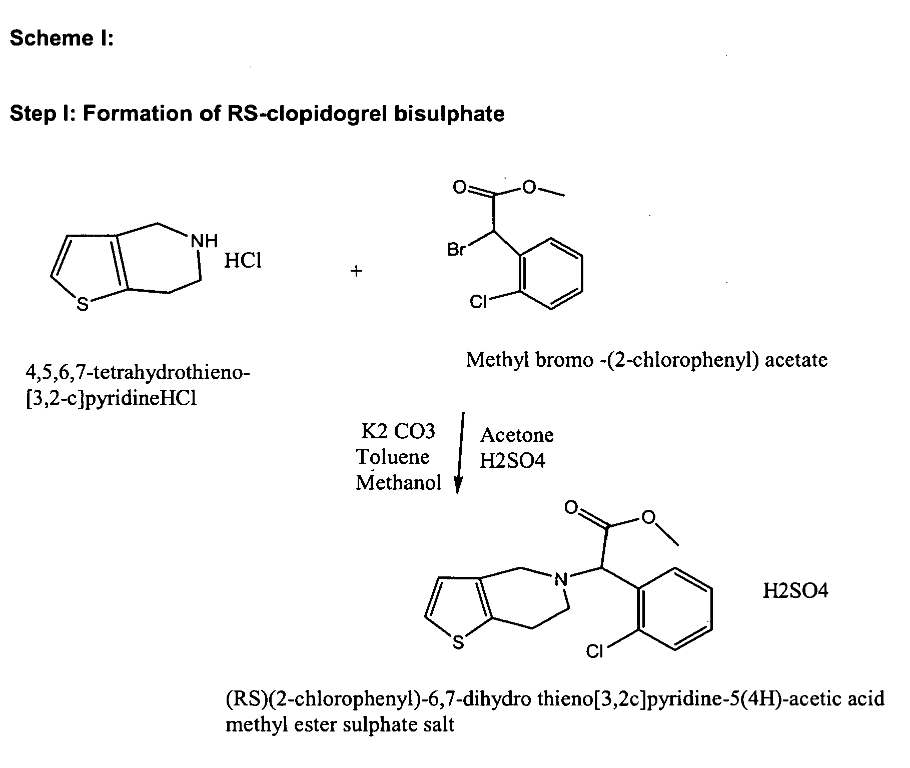

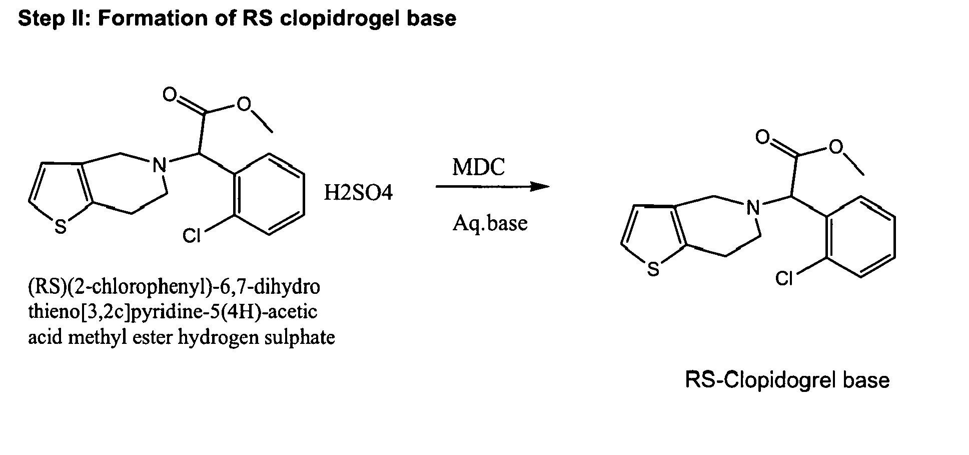

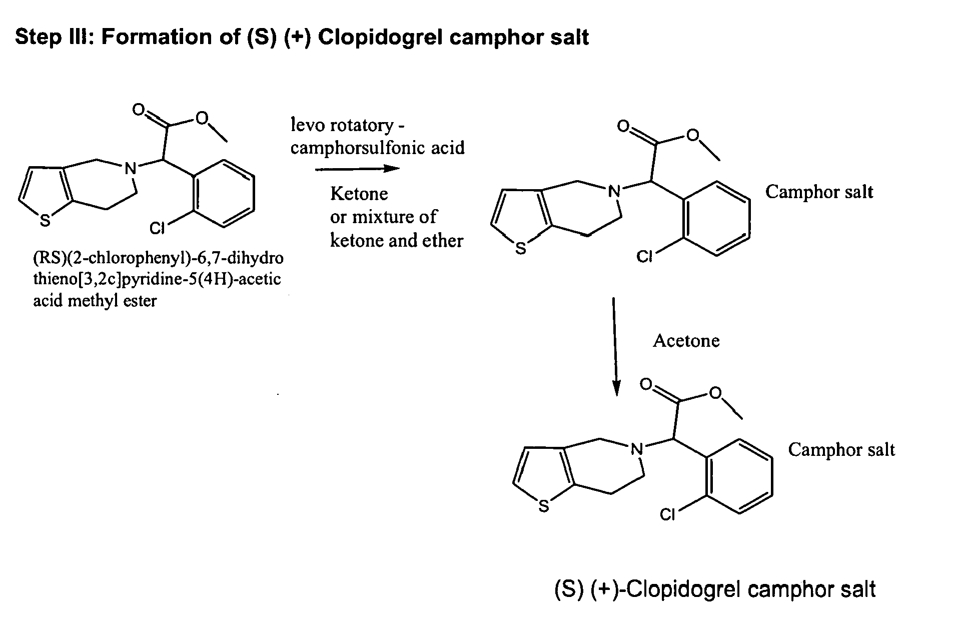

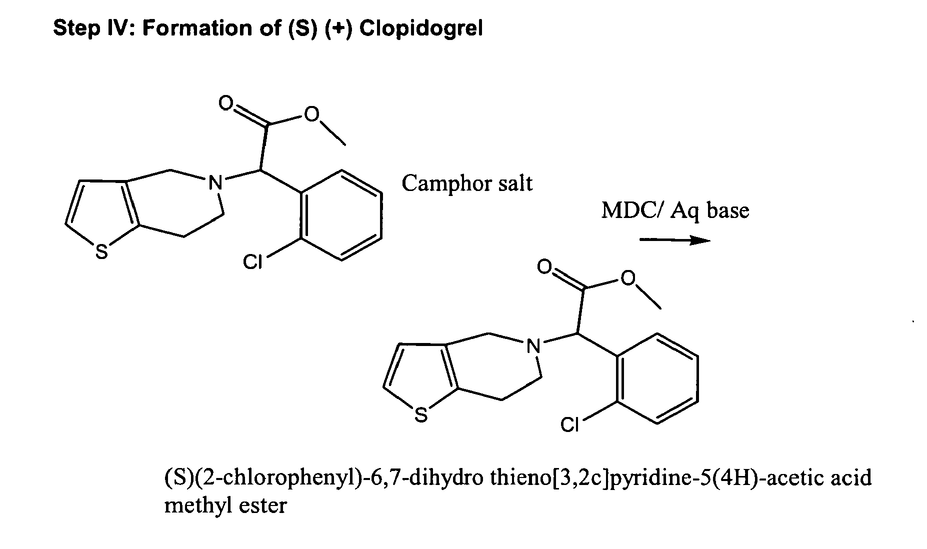

US4847265 discloses racemization of RS clopidogrel to dextro rotatary using levo rotatory camphor sulfonic acid in ketone ( acetone). US6800759 reported the separation of S-isomer as camphor salt using mixture of C₅ to C₁₂ hydrocarbons and co-solvent (DMF/ Toluene)

US20060074242 claims the rapid resolution using levo rotatory camphor sulfonic acid in mixture of solvents ketone and ether at 50°c in 8 hours.

Thus there is need to provide a process for formation of (+) clopidogrel base with fewer steps avoiding formation of intermediate salt like RS Sulphate salt, RS base to save time and make it economical

An object of present invention is to prepare the compound of formula I in its (+) form using simplified and improved process .

Another object of the present invention is to prepare (+) clopidogrel base with out converting in to any other salt forms.

A further object of present invention is to provide a new class of solvent in place of ketone or mixture of solvents ketone and ethers.

Another object of this invention is to provide a quick and simple resolution process with in shortest time of 2 hours and at room temperature.

A further objective of this invention avoiding longer periods and high temperature resolution resulted (s) (+) clopidogrel camphor salt in its purest form and reasonably improved yield.

Another objective of this invention is preparation of (+) clopidogrel base in its purest form of Not Less Than 99.5% with single impurity Not More Than 0.2%.

### Summary of the Invention

According to one aspect of the present invention there is provided a process for the preparation of Methyl (+)-(S)-α-(o-chlorophenyl)-6,7-dihydrothieno-[3,2-c]pyridine-5(4H)acetate of formula I comprising: 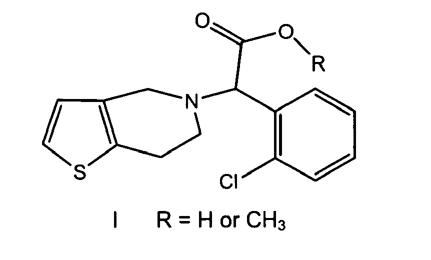
a. reacting 4,5,6,7-tetrahydro[3,2-c]thieno pyridyl methane (II) with 2-halophenyl acetic acid derivative of formula III 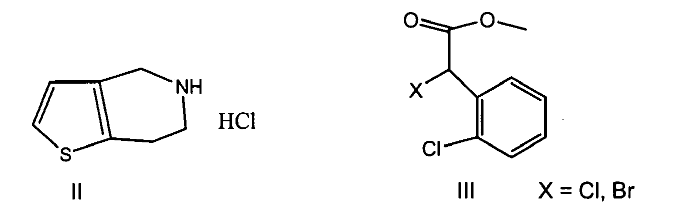
b. extracting the formed product (+/-) clopidogrel in to solvent from the reaction mixture of step a;
c. resolution of (+/-) clopidogrel of step b with levorotatory camphor sulfonic acid and isolation of the (+) clopidogrel camphor sulfonic acid salt;
d. purifying (+) clopidogrel camphor salt
e. neutralization of the camphor salt of step d with aqueous base in solvent and isolation of (+) clopidogrel base by removal of solvent

### Detailed description of the invention:

An objective of the invention is to provide a simplified and modified procedure for the preparation of (+) clopidogrel , by reaction of alpha halo phenyl acetate with (4,5,6,7) tetarhydro thieno pyridine in solvent alcohol or ester or mixture of alcohol and ester.

Resolution of + and - form using levo rotatory camphor sulfonic acid in the same reaction and solvent ester with out isolating either (+/-) clopidogrel or it salt forms.

The present invention provides for the isolation of (+) clopidogrel camphor sulfonic salt and its purification. Purification of (+) clopidogrel camphor salt to its purest form with the impurity level of R-isomer NMT 0.2% using the same ester as solvent.

Isolation of (+) clopidogrel base by neutralizing the camphor salt with aqueous base and extraction to solvent followed by the removal of solvent.

The synthesis is disclosed in scheme II below.

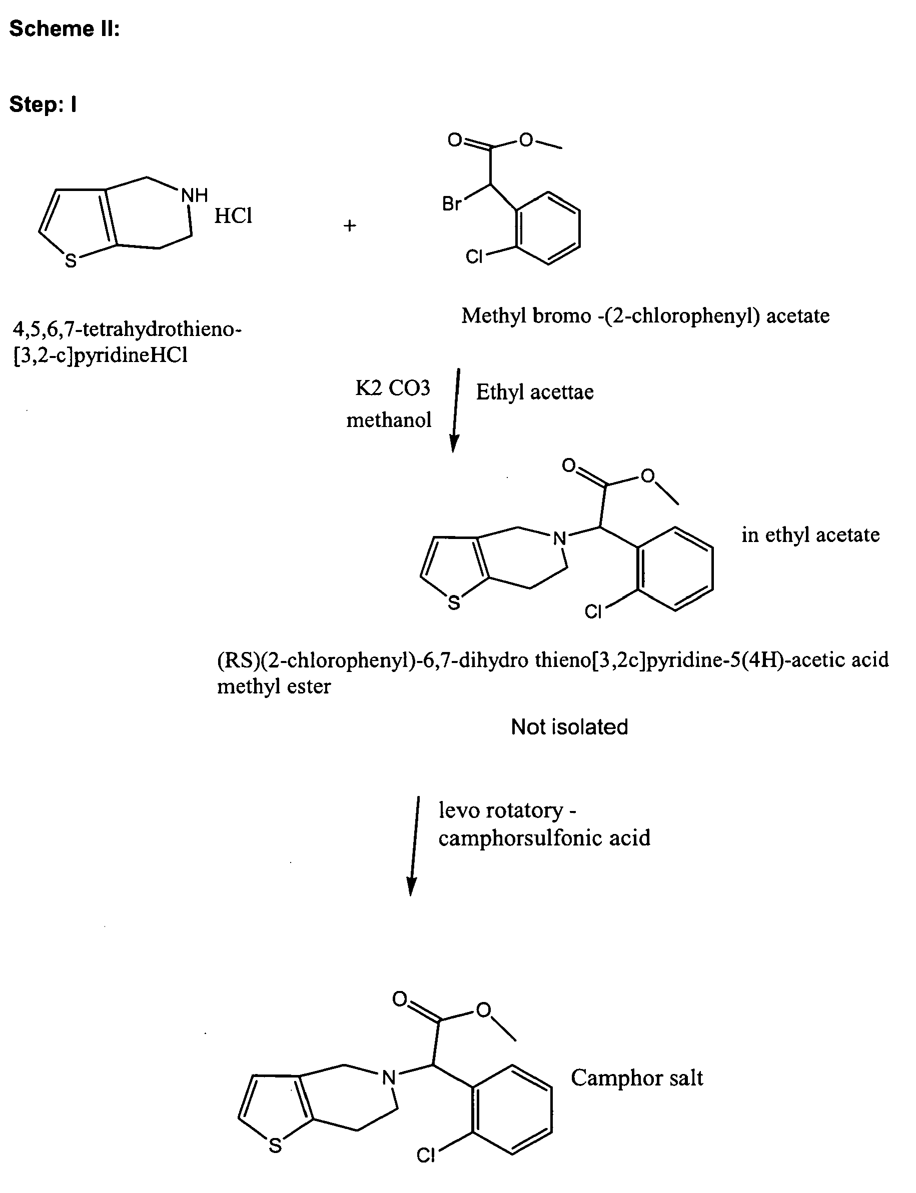

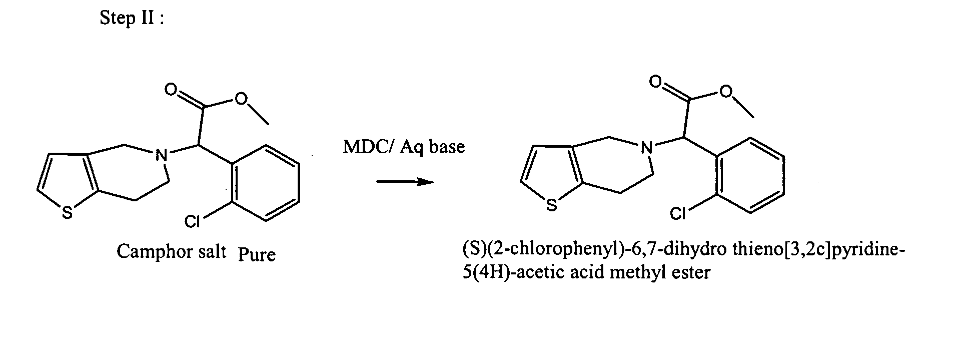

The invention is now described by way of illustrative non-limiting examples.

### Example 1:

### Step 1: Methyl (+)-(s)-α-(o-chlorophenyl)-6,7-dihydrothieno-[3,2-c]pyridine-5(4H)acetate Camphorsulfonic acid salt [(+) clopidogrel camphor sulfonic acid salt]

180 gms ( 0.68 mole) of methyl-2-chloro(2-bromophenyl) acetate is added to a mixture of 120 gms of potassium carbonate and 100 gms ( 0.57 mole) of [4,5,6,7]-tetrahydro thieno[3,2-c]pyridine in 600 ml methanol and 500 ml ethyl acetate. The mass is heated to reflux and refluxed for 4 hours. Mass is cooled to 40 ° C, in organics are filtered and filtrate is diluted with water. Organic layer is separated. Aqueous layer is extracted with ethyl acetate. 65 gms ( 0.28 mole) of levo rotatory camphor sulfonic acid are added to combined organic layer. Reaction mass is maintained at room temperature for two hours, cooled to 0°C and precipitated camphor salt is isolated and purified in ethyl acetate.

Dry weight: - 105 gms HPLC purity : NLT 99.5%. Yield -66.5%.

### Step 2: Methyl(+)-(S)-α-(o-chlorophenyl)-6,7-dihydrothieno-[3,2-c]pyridine-5(4H)-acetate [(+) (S) clopidogrel ]

105 gms (0.19 mole) of pure camphor salt from step 1 is charged in to 600 ml dichloro methane, cooled to 0 ° C and neutralized with aqueous sodium bicarbonate solution. Layers separated aqueous layer is re-extracted with methylene chloride. Combined organic layer dried over sodium sulphate and removed completely under vacuum.

Weight of free base : ~ 60 gms.
Yield : 98.5 % HPLC purity : NLT 99.5%.
Chiral impurity (S-isomer) NMT : 0.20%.

### Example 2:

### Step 1: Methyl (+)-(S)-α-(o-chlorophenyl)-6,7-dihydrothieno-[3,2-c]pyridine-5(4H)acetate Camphorsulfonic acid salt [(+) clopidogrel camphor sulfonic acid salt]

180 gms ( 0.68 mole) of methyl-2-chloro( 2-bromophenyl) acetate is added to a mixture of 120 gms of potassium carbonate and 100 gms (0.57 mole) of [4,5,6,7]-tetrahydro thieno[3,2-c]pyridine HCl in 1000 ml ethyl acetate. The mass is heated to reflux and refluxed for 4 hours. Mass is cooled to 40 ° C, In organics are filtered and filtrate is diluted with water. Organic layer is separated. Aqueous layer is extracted with ethyl acetate. 65 gms ( 0.28 mole) of levo rotatory camphor sulfonic acid are added to combined organic layer. Reaction mass is maintained at room temperature for two hours , cooled to 0c and precipitated camphor salt is isolated and purified in ethyl acetate.

HPLC purity : NLT 99.5%.
Yield -66.0%.

### Step 2: Methyl(+)-(S)-α-(o-chlorophenyl)-6,7-dihydrothieno-[3,2-c]pyridine-5(4H)-acetate [(+) (S) clopidogrel]

105 gms ( 0.19 mole) of pure camphor salt from step 1 is charged in to 600 ml dichloro methane, cooled to 0 ° C and neutralized with aqueous sodium bicarbonate solution. Layers separated aqueous layer is re extracted with methylene chloride. Combined organic layer dried over sodium sulphate and removed completely under vacuum.

Yield : 98 %.
HPLC purity : 99.5%.
Chiral impurity ( S-isomer) NMT : 0.20%.

### Example 3:

### Step 1: Methyl (+)-(S)-α-(o-chlorophenyl)-6,7-dihydrothieno-[3,2-c]pyridine-5(4H)acetate Camphorsulfonic acid salt [(+) clopidogrel camphor sulfonic acid salt]

180 gms ( 0.68 mole) of methyl-2-chloro( 2-bromophenyl) acetate is added to a mixture of 120 gms of potassium carbonate and 100 gms ( 0.57 mole) of [4,5,6,7]-tetrahydro thieno[3,2-c]pyridine HCl in 800 ml methanol. The mass is heated to reflux and refluxed for 4 hours. Mass is cooled to 40 ° C, diluted with water. Extracted with ethyl acetate. Organic layer is separated. Aqueous layer is extracted with ethyl acetate. 65 gms ( 0.28 mole) of levo rotatory camphor sulfonic acid are added to combined organic layer. Reaction mass is maintained at room temperature for two hours, cooled to 0 ° c and precipitated camphor salt is isolated and purified in ethyl acetate.
HPLC purity : NLT 99.5%.
Yield -66.0%.

### Step 2: Methyl(+)-(S)-α-(o-chlorophenyl)-6,7-dihydrothieno-[3,2-c]pyridine-5(4H)-acetate [(+) (S) clopidogrel]

105 gms ( 0.19 mole) of pure camphor salt from step 1 is charged in to 600 ml dichloro methane, cooled to 0 ° C and neutralized with aqueous sodium bicarbonate solution.

Layers separated aqueous layer is re extracted with methylene chloride. Combined organic layer dried over sodium sulphate and removed completely under vacuum.
Yield : 98 %.
HPLC purity: 99.5%.
Chiral impurity ( S-isomer) NMT : 0.20%.

### Example 4:

### Step 1: Methyl (+)-(S)-α-(o-chlorophenyl)-6,7-dihydrothieno-[3,2-c]pyridine-5(4H)acetate Camphorsulfonic acid salt [(+) clopidogrel camphor sulfonic acid salt]

180 gms (0.68 mole) of methyl-2-chloro( 2-bromophenyl) acetate are added to a mixture of 120 gms of potassium carbonate and 100 gms ( 0.57 mole) (4,5,6,7)-tetrahydro thieno[3,2-c]pyridine in 800 ml methanol. The mass is heated to reflux and refluxed for 4 hours. Methanol distilled under vacuum. Mass is cooled to 40 ° C, diluted with water. Ethyl acetate 800 ml is charged and stirred for 30 mins. Organic layer is separated. Aqueous layer is extracted with ethyl acetate. 65 gms ( 0.28 mole) of camphor sulfonic acid is added to combined organic layer. Reaction mass is maintained at room temperature for two hours, cooled to 0°C and precipitated camphor salt is isolated and purified in ethyl acetate.
HPLC purity : NLT 99.5%.
Yield : ∼66%.

### Step 2: Methyl(+)-(S)-α-(o-chlorophenyl)-,6,7-dihydrothieno-[3,2-c]pyridine-5(4H)-acetate [(+) (S) clopidogrel]

110 gms of pure camphor salt from step 1 is charged in to 500 ml dichloro methane, cooled to 0 ° C and neutralized with aqueous sodium bicarbonate solution. Layers separated aqueous layer is re extracted with methylene chloride. Combined organic layer dried over sodium sulphate and removed completely under vacuum.
Yield 98%.

HPLC purity : NLT 99.5%.
Chiral impurity ( S-isomer) NMT : 0.20%.

### Example 5:

### Step 1: Methyl (+)-(S)-α-(o-chlorophenyl)-6,7-dihydrothieno-[3,2-c]pyridine-5(4H)acetate Camphorsulfonic acid salt [(+) clopidogrel camphor sulfonic acid salt]

180 gms of methyl-2-chloro( 2-bromophenyl) acetate are added to a mixture of 120 gms of potassium carbonate and 100 gms (4,5,6,7)-tetrahydro thieno[3,2-c]pyridine HCl in 800 ml methanol. The mass is heated to reflux and refluxed for 4 hours. Methanol distilled under vacuum. Mass is cooled to 40° C, diluted with water. RS clopidogrel base is extracted with ethyl acetate. Layers separated, solvent removed under vacuum. Methyl isobutyl ketone 600 ml is charged in to above free base and stirred for 30 mins. Acetone is added to above solution. 65 gms (0.28 mole) of levo rotatory camphor sulfonic acid are added to above solution. Reaction mass is maintained at room temperature for 15 hours, cooled to 0 ° C and precipitated camphor salt is isolated by filtration. Wet camphor salt is purified in ethyl acetate.
Dry weight: ~100 gms.
HPLC purity : NLT 99.5%.

### Step 2: Methyl(+)-(S)-α-(o-chlorophenyl)-6,7-dihydrothieno-[3,2-c]pyridine-5(4H)-acetate [(+) (S) clopidogrel]

100 gms of pure camphor salt from step 1 is charged in to 500 ml dichloro methane, cooled to 0 ° C and neutralized with aqueous sodium bicarbonate solution. Layers separated aqueous layer is re-extracted with methylene chloride. Combined organic layer dried over sodium sulphate and removed completely under vacuum.

Weight of free base : 58 to 60 gms.
Yield : ~96%.
Single impurity including Chiral impurity ( S-isomer) NMT : 0.20.

### Example 6:

### Step 1: Methyl (+)-(S)-α-(o-chlorophenyl)-6,7-dihydrothieno-[3,2-c]pyridine-5(4H)acetate Camphorsulfonic acid salt [(+) clopidogrel camphor sulfonic acid salt]

180 gms of methyl-2-chloro( 2-bromophenyl) acetate are added to a mixture of 120 gms of potassium carbonate and 100 gms (4,5,6,7)-tetrahydro thieno[3,2-c]pyridine HCl in 800 ml methanol. The mass is heated to reflux and refluxed for 4 hours. Methanol distilled under vacuum. Mass is cooled to 40 ° C, diluted with water. RS clopidogrel base is extracted with ethyl acetate. Layers separated, solvent removed under vacuum. Methyl isobutyl ketone 300 ml is charged in to above free base and stirred for 30 mins. Ethyl acetate 300 ml is added to above solution. 65 gms (0.28 mole) of levo rotatory camphor sulfonic acid are added to above solution. Reaction mass is maintained at room temperature for 15 hours, cooled to 0 ° C and precipitated camphor salt is isolated by filtration. Wet camphor salt is purified in ethyl acetate.
Dry weight: -100 gms.
HPLC purity : NLT 99.5%.

### Step 2: Methyl(+)-(S)-α-(o-chlorophenyl)-6,7-dihydrothieno-[3,2-c]pyridine-5(4H)-acetate [(+) (S) clopidogrel]

100 gms of pure camphor salt from step 1 is charged in to 500 ml dichloro methane, cooled to 0 ° C and neutralized with aqueous sodium bicarbonate solution. Layers separated aqueous layer is re-extracted with methylene chloride. Combined organic layer dried over sodium sulphate and removed completely under vacuum.

Weight of free base : 59 to 60 gms.
Yield : ∼96%.

Single impurity including chiral impurity (S-isomer) NMT : 0.20.

Some preferred embodiments of the invention include the following:
1. A process for the preparation of Methyl (+)-(S)-α-(o-chlorophenyl)-6,7-dihydrothieno-[3,2-c]pyridine-5(4H)acetate of formula I: 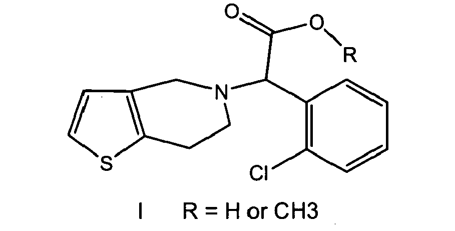 the process comprising:
   a. reacting 4,5,6,7-tetrahydro[3,2-c]thieno pyridyl methane (II) with 2-halophenyl acetic acid derivative of formula III 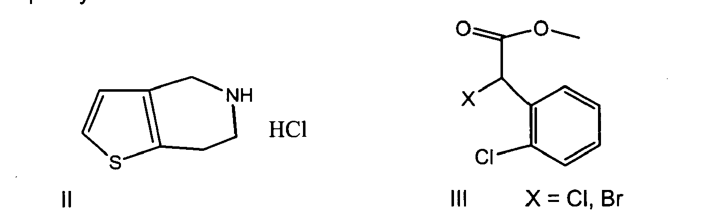
   b. extracting the formed product (+/-) clopidogrel in to solvent from the reaction mixture of step a;
   c. resolution of (+/-) clopidogrel of step b with levorotatory camphor sulfonic acid and isolation of the (+) clopidogrel camphor sulfonic acid salt;
   d. purifying (+) clopidogrel camphor salt; and
   e. neutralization of the camphor salt of step d with aqueous base in solvent and isolation of (+) clopidogrel base by separation and removal solvent.
2. A process according to embodiment 1, wherein steps a, b and c are carried out in single stage with out isolating any R/S clopidogrel or its salt.
3. A process according to embodiment 2, wherein the isolated salt is (S) (+) clopidogrel camphor sulfonic salt.
4. A process according to any one of embodiments 1 to 3, wherein the compound of formula III is selected from 2-bromo-2-(chlorophenyl)acetic acid methyl ester and 2-chloro-2-(chlorophenyl)acetic acid methyl ester.
5. A process according to any one of embodiments 1 to 4, wherein the reaction in step a is carried out in single solvent or mixture of solvents.
6. A process according to embodiment 5, wherein the solvent is selected from alcohol, ester and the mixture of alcohol and ester.
7. A process according to embodiment 6, wherein the alcohol is selected from ethanol and methanol, preferably methanol.
8. A process according to embodiment 6, wherein the solvent ester is selected from methyl and ethyl acetate, preferably ethyl acetate.
9. A process according to any one of embodiments 1 to 8, wherein the reaction in step a is carried out at temperature 25° C to 75 ° C, preferably at 45-60 °C.
10. A process according to any one of embodiments 1 to 9, wherein the solvent for extraction in step b is ethyl acetate.
11. A process according to any one of embodiments 1 to 10, wherein the solvent for in step c resolution is ester.
12. A process according to embodiment 11, wherein the ester is selected from ethyl acetate and methyl acetate, preferably ethyl acetate.
13. A process according to any one of embodiments 1 to 12, wherein the resolution in step c is carried out at room temperature.
14. A process according to embodiment 13, wherein the room temperature ranges from 15 to 50 °C, preferably 25 to 35 °C.
15. A process according to any one of embodiments 1 to 14, wherein the resolution is carried out for the period of 1 to 5 hours, preferably for 2 to 3 hours, more preferably for 2 hours.
16. A process according to any one of embodiments 1 to 15, wherein (S) (+) clopidogrel camphor salt isolated in step c by cooling to 35 to -10 ° c, preferably -5°C to 5 °C, more preferably 0 °C.
17. A process according to any one of embodiments 1 to 16, wherein the resolution reagent in step c levo rotatory camphor Solfonic acid is used.
18. A process according to embodiment 17 wherein camphor sulfonic acid used for resolution is in mole ratio of 0.40 to 0.60 mole for one mole of 4,5,6,7-tetrahydro [3,2-c]thieno pyridine, preferably 0.45 to 0.55 moles for mole of reactant theno derivative, more prefeably 0.50 mole for mole.
19. A process according to any one of embodiments 1 to 18, wherein the solvent for resolution in step c is a mixture of solvents.
20. A process according to embodiment 19 wherein the solvent mixture is selected from ketones and a ketone and ester.
21. A process according to embodiment 20 wherein the mixture of ketones is methyl isobutyl ketone and acetone.
22. A process according to embodiment 20 wherein the solvent mixture of ketone and ester is methyl isobutyl ketone and ethyl acetate.
23. A process according to embodiment 20 wherein the solvent mixture or acetone and ethyl acetate.
24. A process according to any one of embodiments 1 to 23, wherein the solvent for purification of camphor salt in step d is selected from ketone and ester.
25. A process according to embodiment 24 wherein the ketone is acetone
26. A process according to embodiment 24 wherein the ester is ethyl acetate.
27. A process according to any one of embodiments 1 to 26, wherein the base for neutralization in step e is aqueous base.
28. A process according to embodiment 27 wherein aqueous base is selected from sodium hydrogen carbonates, potassium hydrogen carbonates, sodium carbonates, potassium carbonates and sodium hydroxides, potassium hydroxides, more preferably sodium hydrogen carbonate or sodium carbonate.
29. A process according to any one of embodiments 1 to 28, wherein solvent used for extraction of clopidogrel base in step e is selected from methylene chloride and ethyl acetate.
30. A process according to any one of embodiments 1 to 29, wherein the purity of the (+) (S)-clopidogrel in steps a to e is NLT 99.5%.
31. A process according to embodiment 30 wherein the Chiral impurity (R)-Isomer is NMT 0.20%, preferably NMT 0.1 %.
32. A process according to embodiment 31 wherein the individual impurity is NMT 0.20%, preferably NMT 0.1%.

## Claims

1. A process for the preparation of Methyl (+)-(S)-α-(o-chlorophenyl)-6,7-dihydrothieno-[3,2-c]pyridine-5(4H)acetate of formula I: 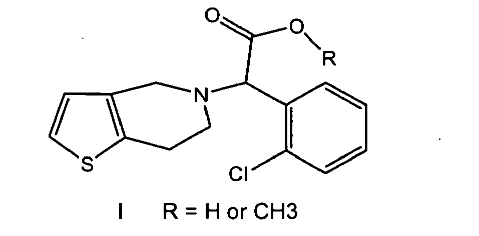 the process comprising:
a. reacting 4,5,6,7-tetrahydro[3,2-c]thieno pyridyl methane (II) with 2-halophenyl acetic acid derivative of formula III: 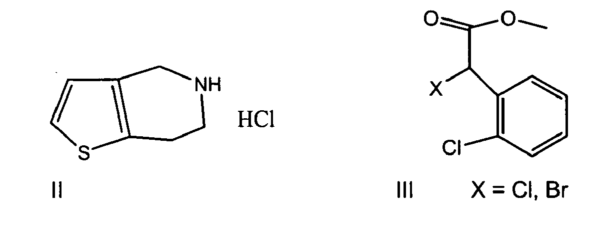
b. extracting the formed product (+/-) clopidogrel in to solvent from the reaction mixture of step a;
c. resolution of (+/-) clopidogrel of step b with levorotatory camphor sulfonic acid and isolation of the (+) clopidogrel camphor sulfonic acid salt;
d. purifying (+) clopidogrel camphor salt; and
e. neutralization of the camphor salt of step d with aqueous base in solvent and isolation of (+) clopidogrel base by separation and removal solvent.

2. A process according to claim 1, wherein steps a, b and c are carried out in single stage without isolating any R/S clopidogrel or its salt.

3. A process according to claim 2, wherein the isolated salt is (S) (+) clopidogrel camphor sulfonic salt.

4. A process according to any one of claims 1 to 3, wherein the compound of formula III is selected from 2-bromo-2-(chlorophenyl)acetic acid methyl ester and 2-chloro-2-(chlorophenyl)acetic acid methyl ester.

5. A process according to any one of claims 1 to 4, wherein the reaction in step a is carried out in single solvent or mixture of solvents.

6. A process according to any one of claims 1 to 5, wherein the reaction in step a is carried out at a temperature from 25° C to 75 ° C.

7. A process according to any one of claims 1 to 6, wherein the solvent for extraction in step b is ethyl acetate.

8. A process according to any one of claims 1 to 7, wherein the solvent for in step c resolution is an ester.

9. A process according to any one of claims 1 to 8, wherein the resolution in step c is carried out at a temperature of from 15 °C to 50 °C.

10. A process according to any one of claims 1 to 9, wherein the resolution is carried out for a period of from 1 to 5 hours.

11. A process according to any one of claims 1 to 10, wherein (S) (+) clopidogrel camphor salt is isolated in step c by cooling to a temperature of from 35 °C to -10°C.

12. A process according to any one of claims 1 to 11, wherein the resolution reagent in step c is levo rotatory camphor sulfonic acid.

13. A process according to claim 12, wherein camphor sulfonic acid used for resolution is in a mole ratio of 0.40 to 0.60 mole for one mole of 4,5,6,7-tetrahydro [3,2-c]thieno pyridine.

14. A process according to any one of claims 1 to 13, wherein the solvent for resolution in step c is a mixture of solvents.

15. A process according to any one of claims 1 to 14, wherein the solvent for purification of the camphor salt in step d is selected from ketone and ester.

16. A process according to any one of claims 1 to 15, wherein the base for neutralization in step e is an aqueous base.

17. A process according to any one of claims 1 to 16, wherein the solvent used for extraction of the clopidogrel base in step e is selected from methylene chloride and ethyl acetate.

18. A process according to any one of claims 1 to 17, wherein the purity of the (+) (S)-clopidogrel in steps a to e is NLT 99.5%.
